## Europäisches Patentamt

## European Patent Office

## Office européen des brevets

(11) Publication number: **0 184 588**
**B1**

---

(12) **EUROPEAN PATENT SPECIFICATION**

(45) Date of publication of patent specification: **08.03.89**

(51) Int. Cl.⁴: **A 61 B 1/26**

(21) Application number: **84115159.0**

(22) Date of filing: **11.12.84**

---

(54) **Laryngoscope with disposable blade and light conductor.**

---

(43) Date of publication of application:
**18.06.86 Bulletin 86/25**

(45) Publication of the grant of the patent:
**08.03.89 Bulletin 89/10**

(84) Designated Contracting States:
**DE FR GB IT SE**

(56) References cited:
**WO-A-83/01373**
**US-A-3 507 272**
**US-A-3 943 920**
**US-A-4 112 933**
**US-A-4 126 127**

(73) Proprietor: **Bauman, Jack**
**1677 San Onofre Drive**
**Pacific Palisades California 90272 (US)**

(72) Inventor: **Bauman, Jack**
**1677 San Onofre Drive**
**Pacific Palisades California 90272 (US)**

(74) Representative: **Howden, Christopher Andrew et al**
**FORRESTER & BOEHMERT Widenmayerstrasse 4/I**
**D-8000 München 22 (DE)**

---

Courier Press, Leamington Spa, England.

EP 0 184 588 B1

## Description

This invention relates generally to medical instruments and more particularly to a laryngoscope having a blade primarily used to facilitate insertion of an endotracheal tube in a patient wherein the blade is disposable.

Most laryngoscopes generally comprise a laryngoscope blade and cooperating handle, both made of metal. These two items are connected together to form a general L-shape. The handle normally serves as an enclosure for batteries for energizing an appropriate light bulb secured adjacent to the blade and connected by wires to the batteries in the handle. This bulb illuminates the patient's mouth and larynx entrance areas. A first surface on the blade itself is used to lift the tongue and mandible of a patient when the patient is in a supine position or depress the tongue and mandible of the patient if the patient is in an upright position. This action prevents the patient's tongue from obstructing the channel of vision.

While the instrument is useful for examining the larynx, its primary function is to expose the larynx in a manner to facilitate the insertion of an endotracheal tube. In this respect, the patient usually is in a supine position on an operating table with his or head extending backwardly. In this position, as described, the first surface of the laryngoscope blade is used to lift the tongue and mandible upwardly to expose the larynx, the light on the blade being positioned beneath the lifting first surface of the blade. A second surface of the blade spaced rearwardly of the first surface is in a position opposing the upper front teeth of the patient.

In using the laryngoscope, there is almost invariably an unconscious tendency to use the upper front teeth of the patient as a fulcrum for the blade in exposing the larynx. Because of the metal construction of the blade, the patient's front teeth often are chipped by such contact and occasionally the teeth may be broken or knocked out.

In addition to the foregoing problems, conventional laryngoscopes should be sterilized after each use, or at a minimum the blade for the laryngoscope must be detached from the handle and sterilized.

In U.S. Patent No. 3,826,248 there is shown an improved laryngoscope blade wherein that portion of the conventional laryngoscope blade defining the referred to second surface opposing the upper front teeth of the patient is removed and a plastic insert substituted for the removed portion. This plastic insert is of elastic material, capable of flexing in a manner to cushion contact with the patient's upper front teeth should the same be used unconsciously as a fulcrum when manipulating the blade to expose the larynx. This laryngoscope blade further carries a light bulb as in a conventional blade. In one embodiment, however, the light bulb is positioned in the foot of the blade and a fiber optic light conductor is used to direct the light.

While the problem of damaging teeth is solved to a large extent by the above described laryngoscope blade, there still remain problems of added expense in the actual manufacture of the blade when compound materials are used and a light bulb is incorporated in the blade.

U.S. Pat. 4,295,465 shows another type blade for a laryngoscope including a flange mechanically pivoted to part of the blade and biased by a spring. This flange is positioned to engage the patient's teeth and cushion action of the blade against the teeth when the teeth are used as a fulcrum.

In my prior U.S. Design Pat. No. 242,396, I disclose a unique shaping and contouring for a disposable cover to be used to cover a laryngoscope blade. In U.S. Pat. 4,037,588 there is shown a disposable laryngoscope blade made entirely of plastic, this plastic material being capable of optically conducting light so that a light bulb can be arranged in the handle juxtaposed to a surface of the plastic blade. In this blade construction, in order to provide proper strength for the portion of the blade that depresses the tongue, the material has had to be fairly thick. As a consequence, the second surface portion opposing the patient's teeth corresponding to that described for conventional blades is also relatively rigid and thus the problem of damaging the patient's teeth has not been solved by this all plastic blade member.

All of the foregoing is the closest prior art of which I am aware to my present invention.

With the foregoing in mind, the present invention contemplates an improved laryngoscope in which several of the advantages of prior art laryngoscopes are incorporated without attendant disadvantages.

According to one aspect of the present invention, there is provided a non-metallic laryngoscope blade formed from a plastic material into a unitary structure and adapted to be detachably connected to a handle in a generally L-shaped configuration, said blade comprising:

(a) a lower tongue engaging flange section;

(b) an upper teeth engaging flange section; and

(c) an upstanding web interconnecting the upper and lower flanges and formed integral therewith, characterised in that said web is provided with one or more areas of weakness which provide the web with sufficient flexibility so that when the upper teeth engaging flange is urged into contact with a patient's larynx, the web will flex or bend, thereby avoiding damage to the patient's teeth.

According to another aspect of the invention, there is provided a laryngoscope including in combination:

(a) an elongated handle provided with a light source at the proximal end thereof;

(b) a detachable non-metallic blade formed from a plastic material into a unitary structure comprising a lower tongue engaging flange section, an upper teeth engaging flange section, and an upstanding web interconnecting the upper and lower flanges and formed integral therewith, and

(c) means coupling the blade to the proximal end of the handle in generally L-shaped configuration, characterised in that said web is provided with one or more areas of weakness which provide the web with sufficient flexibility so that when the upper teeth engaging flange is urged into contact with a patient's teeth during examination of a patient's larynx, the web will flex or bend, thereby avoiding damage to the patient's teeth.

More particularly, a preferred form of laryngoscope, includes an elongated handle and a disposable blade embodying the invention with a light conductor held by the material of the blade, this light conductor being made of high quality light transmissive material such as clear plastic. By utilising a separate light conductor rather than using the non-metallic or plastic material of the blade itself as a light conductor, there is removed any limitations on the particular type of plastic used for the blade and accordingly, an appropriate flexible portion may be formed for cushioning the blade portion normally opposed to the patient's teeth, thereby minimizing damage to the patient's teeth.

In this preferred form of laryngoscope, a light bulb is incorporated in the handle of the laryngoscope. However, the mechanism for automatically energizing the light bulb is greatly simplified over prior art arrangements.

The blade itself is detachable from the handle along with the light conductor so that the same is simply disposed of after use so that repeat sterilization is unnecessary.

An embodiment of the invention is described below with reference to the accompanying drawings in which:

FIGURE 1 is a side elevational view of a laryngoscope embodying this invention preparatory to being used on a patient in a supine position;

FIGURE 2 is a greatly enlarged ¾ rear perspective view of the laryngoscope of Figure 1 showing certain portions in exploded relationship;

FIGURE 3 is a fragmentary cross section of a portion of the laryngoscope of Figure 2;

FIGURE 4 is a side elevational view of the laryngoscope blade of Figure 2 showing a component in exploded relationship thereto;

FIGURE 5 is a fragmentary elevational view showing a portion of the opposite side of the blade of Figure 4;

FIGURE 6 is a greatly enlarged cross section taken in the direction of the arrows 6-6 of Figure 4;

FIGURE 7 is a side elevational view of a modified blade illustrating an optional feature of this invention;

FIGURE 8 is a cross section taken in the direction of the arrow 8-8 of Figure 7;

FIGURE 9 is a side elevational view of yet another blade incorporating a still further alternative feature in accordance with the present invention; and

FIGURE 10 is a cross section taken in the direction of the arrows 10-10 of Figure 9.

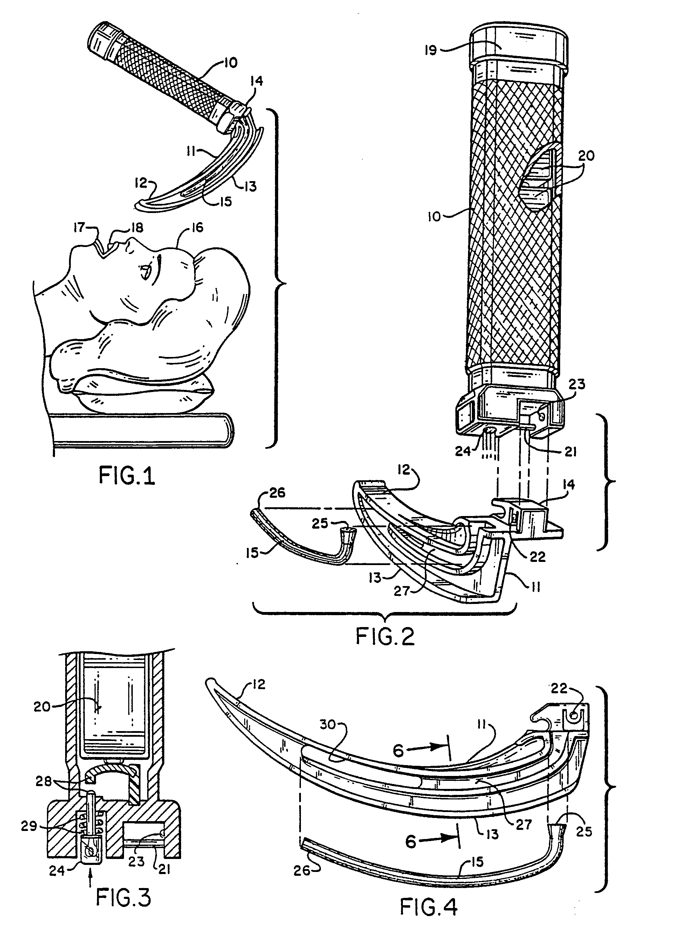

Referring first to Figure 1 the laryngoscope includes an elongated handle 10. A disposable blade 11 of non-metallic material includes a tongue engaging portion 12 and a flexible portion 13 normally in a position opposing a patient's teeth when the blade is in use. Means 14 are provided coupling the blade to one end of the handle 10 for movement from a folded position to an operative position in which the blade defines with the handle a general L-shape.

A light conductor 15 which might comprise clear plastic is held in the blade with a light entrance end positioned to be in optically coupling relationship with an appropriate light source in the one end of the handle 10 when the blade assumes its operative position shown. The other end of the light conductor 15 is positioned so that light from the source in the handle exits along the line of sight of the blade in a direction to illuminate a patient's larynx when the blade is in use.

Thus, still referring to FIGURE 1, there is shown a patient 16 in a supine position. The tongue engaging portion 12 of the blade 11 is arranged to depress the patient's tongue and mandible indicated at 17. When the blade is so used, and as described heretofore, there is an unconscious tendency to use the patient's upper teeth indicated at 18 as a fulcrum for the blade, these teeth being engaged by the portion 13 of the blade 11. By making this portion flexible as will be described in greater detail as the description proceeds, the risk of damaging the patient's teeth when utilizing the blade of the present invention is minimized.

It will be understood that because the blade itself is of non-metallic material, it is fairly economical to manufacture. Further, since the light bulb or light source is incorporated in the handle 10, the blade along with the light conductor can be detached from the handle and disposed of after use so that repeated sterilization is unnecessary.

Referring now to FIGURE 2, the foregoing as well as further features and advantages of this invention will become evident.

Referring first to the handle 10, it will be noted that the same includes a removable cap 19 at its upper end opposite the one end coupled to the blade 11, for receiving batteries 20.

The coupling means 14 described briefly in FIGURE 1 is conventional in certain respects. Thus, the means 14 includes an appropriate cross groove for receiving a pivot rod 21 in the one end of the handle 10. Cooperating dimples and ball detents 22 and 23 may be provided to hold the blade 11 in its operative position wherein it assumes the L-shape configuration shown in FIGURE 1.

The light source heretofore referred to in the handle 10 includes a light bulb 24 arranged to project slightly from the undersurface of the one end of the handle 10. Essentially, the light bulb 24 is juxtaposed the entrance end of the light conductor 15 described in FIGURE 1. This entrance end is indicated in the exploded view of the light conductor in FIGURE 2 at 25. The opposite end of the light conductor 15 from which light exits is shown at 26.

The light conductor 15 shown in FIGURE 2 is held to the blade 11 by an appropriate channel 27 formed in a side of the blade immediately beneath the tongue engaging portion 12 of the blade.

Referring to FIGURE 3, it will be noted that the one end of the handle includes contact means 28 engaged by the light conductor entrance end 25 bearing against the bulb 24 to urge it upwardly as viewed in FIGURE 3 when the blade is coupled about the pivot rod 21 and swung into its operative position. In other words, the light bulb 24 is automatically energized when the blade is folded outwardly to its operative position by action of depressing the bulb 24. In this respect, a small spring 29 would normally bias the bulb 24 downwardly as viewed in FIGURE 3 to maintain the contact mans 28 open so that the bulb is normally deenergized and will only be energized when the blade is swung to its operative position.

The foregoing feature is important in that it eliminates the necessity for any type of auxiliary switch. Further, the spring 29 biasing the bulb 24 against the light entrance portion 25 of the light conductor 15 assures a reliable and efficient optical coupling of the light from the bulb into the entrance end of the light conductor.

Referring now to FIGURE 4, there is again shown the blade 11 in side elevation with the light conductor 15 exploded away. As will be clear, the channel 27 terminates as it approaches the far end of the blade; that is, the left end as viewed in FIGURE 4 in an elongated opening 30. This opening is arranged to receive the far end 26 of the light conductor 15 so that light actually exits from this end on the opposite side of the blade as viewed in FIGURE 4. This light is directed along the line of sight of the blade as viewed by the doctor for proper illumination of the patient's larynx.

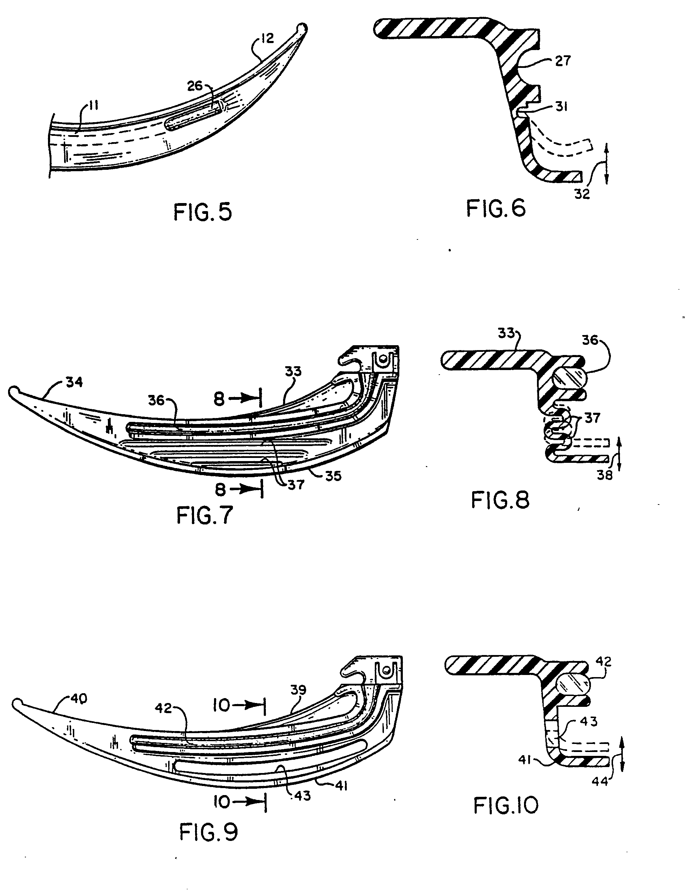

FIGURE 5 shows a fragmentary view of the opposite end of the blade described in FIGURE 4 wherein the light conductor 15 is positioned in the blade with its exit end 26 extending through the opening 30. This exit end 26 after having been passed through the opening 30 of the blade is clearly visible in the showing of FIGURE 5.

Referring to the enlarged cross section of FIGURE 6, the cradle or channel 27 for the light conductor 15 is clearly illustrated. Further, it will be noted that the flexible portion 13 normally opposed to the patient's teeth is rendered flexible in the specific embodiment disclosed by making a longitudinal groove 31 in the blade running between the light conductor 15 when the same is in position and the portion of the blade opposing the patient's teeth. By providing such a groove, there is essentially formed a thinned part of the blade as viewed in cross section permitting flexing of the portion opposing the patient's teeth as indicated by the double headed arrow 32 in FIGURE 6, the flexing taking place between the solid and dotted line positions of the flexible portion 13.

Referring to FIGURE 7, there is shown another blade somewhat straighter than the blade 11 described in FIGURE 4. In this respect, it should be understood that the present invention is applicable to fully straight type blades as well as the curved blades shown.

In FIGURE 7, the blade 33 again includes a tongue engaging portion 34 and a flexible portion 35 normally opposing the patient's teeth. Again, there is provided a light conductor 36 held by the blade in a manner similar to the light conductor 15 described in FIGURE 4.

Rather than a groove such as described at 31 to provide flexibility to the portion 35, there is shown in FIGURE 7 accordion pleats 37.

Referring to the cross section of FIGURE 8, these accordion pleats 37 are more evident and will permit a flexing of the portion 35 opposing the patient's teeth in an up and down direction as viewed in FIGURE 8 and as indicated by the double headed arrow 38.

FIGURE 9 shows yet another blade 39 having a tongue engaging portion 40 and a flexible portion 41 opposing a patient's teeth. Also shown is a light conductor 42 held by the blade 39 in a manner similar to that described for the light conductor 15 of FIGURE 4.

In the embodiment of FIGURE 9, flexibility is imparted to the portion 41 by forming an elongated window in the blade adjacent to the portion 41 opposing the patient's teeth.

Referring to the cross section of FIGURE 10, this window 43 will permit flexing of the portion 41 in an up and down direction as indicated by the double headed arrow 44 as viewed in FIGURE 10.

The embodiments of FIGURES 7 through 10 are set forth merely to indicate that this invention is not limited to any one specific means for providing the desired flexibility of the portion of the blade opposing the patient's teeth.

Operation

In the initial manufacture of the blade described herein, there are provided two molds. One for the blade 11 and one for the light conductor 15. After each piece is molded, it is a simple matter to fit the conductor 15 into the channel 27 of the blade to be held thereby.

With respect to the foregoing, a great advantage is realized by using two separate components and then placing them together. First, there is no limitation as to the type of plastic which can be used for the blade 11. In other words, it is not necessary that this plastic have any particular light transmissive characteristics since the light is transmitted by the light conductor 15 and does not depend on the transmissive characteristics of the plastic for the blade. This advantage means that appropriate plastic material which can be properly worked to provide the desired rigidity for the blade portion engaging the patient's tongue and mandible and the desired flexibility for the blade portion opposing the patient's teeth can readily be realized.

The material for the light conductor 15, on the other hand, can be selected from the highest quality light transmissive clear plastics.

Once the blade and light conductor are assembled, they are appropriately sterilized and packaged.

When the blade is to be used, it is simply removed from the packaging and the means 14 hooked onto the pivot rod 21 described in FIGURE 2, the blade then being swung outwardly from the handle to assume its L-shaped position described in FIGURE 1. This action automatically results in the light entrance end of the clear plastic shown at 25 engaging the end of the bulb 24 described in FIGURE 3 to depress the same against the action of the spring 29. When the ball detents 23 and cooperating dimples 22 register, the bulb 24 is fully depressed so as to close the contacts 28 and energize the bulb. A tight optical contact between the bulb and entrance end of the light conductor is assured as described heretofore.

After the doctor completes his examination or has successfully inserted an endotracheal tube, the blade 11 along with the light conductor is simply decoupled from the handle and thrown away. Inexpensive plastic material exhibiting the desired rigidity and flexibility at various portions can be used for the blade since the light transmissive characteristics of the blade plastic are not a consideration as described. Thus the blade can be economically manufactured and since it does not incorporate a bulb itself, the disposal of the blade after a single use does not pose any serious economic problem.

As already described, the features of this invention can be incorporated on straight type laryngoscope blades and on smaller versions used with children.

It should be understood that the flexible portion can be formed by simply thinning the material in the area involved in place of or in addition to the provision of the groove or accordion pleating or window. For example, the flexible portion in FIGURE 6 is shown as tapering below the groove 31 towards a thinner configuration than the upper portion of the blade used to depress the patient's tongue.

Further, the light conductor can be provided with an exterior coating except at its light entrance and exit ends, to minimize light scattering.

The present invention, accordingly, is not to be thought of as limited to the exact blade shapes and structures described merely for illustrative purposes.

## Claims

1. A non-metallic laryngoscope blade (11) formed from a plastic material into a unitary structure and adapted to be detachably connected to a handle (10) in a generally L-shaped configuration, said blade (11) comprising:

(a) a lower tongue engaging flange section (12);

(b) an upper teeth engaging flange section (13); and

(c) an upstanding web interconnecting the upper and lower flanges (12, 13) and formed integral therewith, characterised in that said web is provided with one or more areas of weakness which provide the web with sufficient flexibility so that when the upper teeth engaging flange (13) is urged into contact with a patient's larynx, the web will flex or bend, thereby avoiding damage to the patient's teeth.

2. The laryngoscope blade of claim 1, wherein the upstanding web is weakened in at least one area by a longitudinal groove (31, Figure 6).

3. The laryngoscope of claim 1 wherein the upstanding web is weakened in at least one area by accordion pleats (37, Figures 7 and 8).

4. The laryngoscope blade of claim 1, wherein the upstanding web is weakened in at least one area by an elongated window.

5. The laryngoscope blade of claim 1, wherein the upstanding web is weakened in at least one area by a thinned webbed section.

6. A laryngoscope including in combination:

(a) an elongated handle (10) provided with a light source (24) at the proximal end thereof;

(b) a detachable non-metallic blade (11) formed from a plastic material into a unitary structure comprising a lower tongue engaging flange section (12), an upper teeth engaging flange section (13), and an upstanding web interconnecting the upper and lower flanges (12, 13) and formed integral therewith, and

(c) means (14, 21) coupling the blade (11) to the proximal end of the handle in generally L-shaped configuration, characterised in that said web is provided with one or more areas of weakness which provide the web with sufficient flexibility so that when the upper teeth engaging flange (13) is urged into contact with a patient's teeth during examination of a patient's larynx, the web will flex or bend, thereby avoiding damage to the patient's teeth.

7. The laryngoscope of claim 5, wherein the blade (11) is provided with a light conductor (13) to conduct light from the light source (24) in the handle to the distal end of the blade (11).

## Patentansprüche

1. Nicht-metallischer Kehlkopfspiegelspatel (11), der aus einem Kunststoff einstuckig ausgebildet ist und zur lösbaren Verbindung mit einem Griff (10) in eine im wesentlichen L-förmige Ausgestaltung eingerichtet ist, wobei der Spatel (11) aufweist:

a) einen auf die Zunge wirkenden Flanschabschnitt (12);

b) einen auf die oberen Zähne wirkenden Flanschabschnitt (13); und

c) einen den oberen und den unteren Flansch (12, 13) miteinander verbindenden und einstückig mit diesen ausgebildeten Steg, dadurch gekennzeichnet, daß der Steg mit einer oder mehreren Schwächungszonen versehen ist, die den Steg ausreichend flexibel machen, so daß bei Aufbringen des auf die oberen Zähne wirkenden Flansches (13) auf den Kehlkopf des patienten der Steg nachgibt oder abbiegt, wodurch eine Beschädigung der Zähne des Patienten vermieden wird.

2. Kehlkopfspiegelspatel nach Anspruch 1, wobei der aufrechte Steg wenigstens in einem Bereich durch eine longitudinale Kerbe (31, Fig. 6) geschwächt wird.

3. Kehlkopfspiegel nach Anspruch 1, wobei der aufrechte Steg in wenigstens einem Bereich durch entsprechende Faltungen (37, Fig. 7 und 8) geschwächt wird.

4. Kehlkopfspiegelspatel nach Anspruch 1, wobei der aufrechte Steg in wenigstens einem Bereich durch ein längliches Fenster geschwächt ist.

5. Kehlkopfspiegelspatel nach Anspruch 1, wobei der aufrechte Steg in wenigstens einem Bereich durch einen Abschnitt mit geringerem Querschnitt gebildet wird.

6. Kehlkopfspiegel, der in Kombination aufweist:

a) einen länglichen Griff (10), der an seinem proximalen Ende mit einer Lichtquelle (24) versehen ist;

b) einen lösbaren, nicht-metallischen Spatel (11), der aus einem Kunststoff einstückig ausgebildet ist mit einem auf die Zunge wirkenden Flanschabschnitt (12), einem auf die oberen Zähne wirkenden Flanschabschnitt (13) und einem aufrechten Steg, der den oberen und den unteren Flansch (12, 13) miteinander verbindet und mit diesem einstückig ausgebildet ist,

c) Mittel (14, 21) zum Ankoppeln des Spatels (11) an das proximale Ende des Handgriffs in einer im wesentlichen L-förmigen Ausbildung, dadurch gekennzeichnet, daß der Steg mit einer oder mehreren Schwächezonen versehen ist, die den Steg mit einer ausreichenden Flexibilität versehen, so daß bei einem Inkontaktbringen des auf die oberen Zähne wirkenden Flansches mit den Zähnen eines Patienten während der Untersuchung des Kehlkopfes des Patienten der Steg nachgibt oder abbiegt, wodurch eine Beschädigung der Zähne des Patienten vermieden wird.

7. Kehlkopfspiegel nach Anspruch 5, wobei der Spatel (11) mit einem Lichtleiter (13) versehen ist, um Licht von einer Lichtquelle (24) in dem Handgriff zu dem distalen Ende des Spatels (11) zu führen.

**Revendications**

1. Spatule de laryngoscope non métallique (11) constituée en un matériau plastique, de structure unitaire et adaptée à être reliée de façon amovible à une poignée (10) sous une configuration générale en forme de L, ladite spatule (11) comprenant:

(a) une section inférieure de contact avec la langue (12);

(b) une section supérieure de contact avec les dents (1); et

(c) un voile vertical interconnectant les sections supérieure et inférieure (12, 13) et formé d'un seul tenant avec elles, caractérisé en ce que ledit voile est muni d'une ou plusieurs régions d'affaiblissement communiquant au voile une flexibilité suffisante pour que lorsque la section de contact avec les dents supérieures (13) est sollicitée en contact avec le larynx du patient, le voile s'infléchisse ou se courbe, évitant ainsi des dégâts aux dents du patient.

2. Spatule de laryngoscope selon la revendication 1, dans lequel le voile vertical est affaibli dans au moins une région par une gorge longitudinale (31, figure 6).

3. Spatule de laryngoscope selon la revendication 1, dans lequel le voile vertical est affaibli dans au moins une région par des plis en accordéon (37, figures 7 et 8).

4. Spatule de laryngoscope selon la revendication 1, dans lequel le voile vertical est affaibli dans au moins une région par une fenêtre allongée.

5. Spatule de laryngoscope selon la revendication 1, dans lequel le voile vertical est affaibli dans au moins une région par une section de voile affaiblie.

6. Laryngoscope comprenant en combinaison:

(a) une poignée allongée (10) munie d'une source lumineuse (24) à son extrémité proximale;

(b) une spatule non métallique amovible (11) constituée en une matière plastique et selon une structure unitaire, comprenant une section inférieure de contact avec la langue (12), une section supérieure de contact avec les dents (13), et un voile vertical interconnectant les sections supérieure et inférieure (12, 13) et formé d'un seul tenant avec elles, et

(c) des moyens (14, 21) reliant la spatule (11) à l'extrémité proximale de la poignée selon une configuration générale en L, caractérisé en ce que ledit voile est muni d'une ou plusieurs régions d'affaiblissement qui communiquent au voile une flexibilité suffisante pour que lorsque la section supérieure de contact avec les dents (13) est sollicitée en contact avec les dents d'un patient pendant l'examen du larynx du patient, le voile s'infléchisse ou se courbe, évitant ainsi des dégâts aux dents du patient.

7. Laryngoscope selon la revendication 5, dans lequel la spatule (11) est munie d'un conducteur de lumière (13) pour diriger la lumière depuis la source de lumière (24) dans la poignée jusqu'à l'extrémité distale de la spatule (11).

FIG.1

FIG.2

FIG.3

FIG.4

1

FIG.5

FIG.6

FIG.7

FIG.8

FIG.9

FIG.10

2